# EUROPEAN PATENT SPECIFICATION

(11) **EP 1 520 546 B1**
(45) Date of publication and mention of the grant of the patent: **02.01.2008**
(21) Application number: 01955764.4
(22) Date of filing: 25.07.2001
(51) Int. Cl.: A61B 18/20

(54) **APPARATUS FOR LASER DEPILATION**
LASEREPILATIONSGERÄT
APPAREIL D'EPILATION LASER

(30) Priority: 12.09.2000 RU 2000123242
(43) Date of publication of application: 06.04.2005
(73) Proprietor: Khomchenko, Vladimir Valentinovich, Moscow 123362 (RU)
(72) Inventor: Khomchenko, Vladimir Valentinovich, Moscow 123362 (RU)
(74) Representative: Jeck, Anton
(86) International application number: PCT/RU2001/000303
(87) International publication number: WO 2002/022034

(56) References cited:
- RU-A- 96 114 909
- US-A- 5 647 866
- US-A- 5 836 938

## Description

### Field of Invention

The subject invention relates to laser medicine, in particular to cosmetology and may be used for hair depilation.

### Background Art

There are several methods of laser depilation which are widely known, namely those based upon the irradiation of skin sections having unwanted hair by single long pulses of ruby lasers (US patent No. 5059192, publication date 22.10.1991, "Method of hair depilation"), of alexandrite lasers (International application No. 9722384, publication date 26.06.1997, "Hair removal by selective photothermolysis with an alexandrite laser"), of neodymium lasers (US patent No. 6050991, publication date 18.04.2000., "Pulsed-emission laser for use in the medical field") and of diode lasers (US patent No. 6027495, publication date 20.03.1997, "Method and apparatus for dermatology treatment").

The described methods are based on the process of melanin coagulation being a part of a hair shaft and a hair follicle. As a result of such irradiation with energy density in the order of 10 - 50 Joules/cm² the coagulation of melanin takes place and, consequently, the thermal destruction of a hair structure.

The above methods envisage the use of single long pulses from 500 microseconds to 30 ms. It is conditioned by the fact that the time of thermal relaxation (TTR) of a hair, i.e. the time during which the hair succeeds to transfer a substantial amount of heat to the surrounding tissue constitutes units of milliseconds. As a result, when using longer single pulses the heat from hair will be transferred to surrounding tissue. In case of using shorter pulses the time of exposure becomes commensurable with the TTR of skin what may lead to its overheating and coagulation. Therefore, in order to achieve effective hair heating up and to minimize the heating up of surrounding tissue it is necessary to apply radiation during the time period commensurate with the time of thermal relaxation.

Among the drawbacks of the above-mentioned methods it is necessary to point out that the mechanism of a hair destruction is based upon the absorption of the radiation by melanin. As a result the methods could be effectively employed only for the patients with fair skin and dark hair. When using the said method with dark-complexioned patients the absorption of radiation by melanin takes place and, as a result, the depigmentation of the considered skin sections. Moreover, as the part of radiation is consumed by absorption in the upper pigmented skin layer, the hair receives less energy than it is necessary for its coagulation.

There are known methods of laser hair depilation using special substances - photosensitizers the ability of which to absorb laser radiation of the definite wavelength is much higher than that of surrounding tissue. In the said methods ruby or YAG:Nd lasers are used with pulse duration of units or ten nanoseconds. The photosensitizer is applied to the skin section having unwanted hair and its particles penetrate the hair canals and envelope the latter (US patent No. 5925035, publication date 20.07.1999., "Hair removal method").

The photosensitizers that selectively dye the hair structure are also used (US patent No. 6063074, publication date 16.05.2000, "Hair removal using a contaminant matched to a laser").
By exerting influence on skin surface with short-pulsed laser radiation of nanosecond duration and the wavelength corresponding to the maximum absorption spectrum of the chosen photosensitizer a selected heating of the photosensitizer is assured and the effect of microexplosions in its particles is achieved. As a result of microexplosions an acoustic wave is produced which, being disseminated along the hair, provokes the mechanical destruction of its structures. Besides, the acoustic wave exerting influence on the photosensitizer's particles contributes to its deeper penetration to the hair canal. As a result, the next radiation pulse is able to destruct the hair section situated deeper.

The said methods are applicable for the dark-complexioned patients and allow to lessen the thermal destruction of surrounding soft tissue but not to eliminate it as the known photosensitizers do not possess sufficiently high selective influence. They envelope or stain not only the hair structures but also surrounding soft tissue causing its undesirable ability to absorb the selected laser radiation.

To the shortcomings of the said methods it is necessary to ascribe that the photosensitizers are not able to penetrate the skin to the full extent of the hair length and, consequently, cannot fully destroy the hair. The exception constitutes only superficial hair with shallow rooting. Moreover, the energy of microexplosions originated within the photosensitizer is distributed equally both to the hair structure and the surrounding soft tissue what causes its mechanical damage. Besides, the photosensitizers are often allergenic.

The most close to the claimed apparatus is the apparatus LIGHTBEAM of the firm "ETOLLE", Italy ("Aesthetics News", No.1, 1999, p.81, Leader in beauty technology). It applies single short laser pulses of a YAG:Nd laser of nanosecond duration and energy density of 10 Joules/cm².

Also US 5 647 866 discloses a similar apparatus.

The said short-pulsed method does not lead to overheating of surrounding soft tissue as when using the nanosecond pulses the influence time appears to be substantially less than the TTR of the hair. Besides, when using the said pulses durations the absorption spectrum of substances undergo significant changes as the energies' densities achieve such values at which the processes described by non-linear optics begin to take place.

As a result, the YAG:Nd laser radiation is effectively absorbed by hair structures and as the pulses duration constitutes only units of nanoseconds that leads to formation of microexplosions which, in turn, provoke acoustic waves bringing about mechanical destruction of hair structures As the effectiveness of radiation absorption by hair structures in the said method increases there appears the possibility of using substantially lesser energy densities of radiation for laser depilation.

However, the use of the said method is limited to the destruction of superficial hair structures. The reason of this is that a single laser pulse is able to provoke a limited number of microexplosions while these explosions will be located in upper layers of the hair. In order to increase the number of microexplosions and the depth of radiation penetration to soft tissue the laser pulse energy is correspondingly increased. But in this case not only hair structures effectively absorb the radiation, but also the surrounding soft tissue. As a result, on one hand, the energy affecting the hair is decreased due to radiation absorption by upper skin layers and, by consequence, the depilation effectiveness becomes reduced, and on the other hand, the soft tissue is destroyed what diminishes the method's selectiveness.

### Disclosure of Invention

The invention is aimed to solve the problem of increasing the effectiveness of depilation with simultaneous reduction of overheating and mechanical damage of surrounding tissue.

The said problem is solved so that by the apparatus for laser depilation pulsed laser radiation of nanosecond duration *in contradistinction* to the known method the radiation is applied in the form of a packet of pulses with energy density from 3 to 60 Joules/cm² and packet duration from 0,5 to 10 ms.

The packet of pulses at that is being formed of at least 5 subsequent pulses.

Besides, the hair is irradiated at least by one packet.

When applying radiation in the form of a packet of pulses the energy density of each single pulse is less than the threshold of skin destruction. Due to the use of pulses of nanosecond duration the effectiveness of radiation absorption by hair structures increases. But as in the case of the prototype the first pulse damages only the superficial hair layers.

In order to increase the depth of radiation penetration to the soft tissue and subsequent destruction of deeper hair layers the first pulse is followed by the next pulse of a packet. This pulse is able to penetrate deeper as the superficial absorbing centers have already been destroyed by the first pulse of the packet.

Each subsequent pulse of the packet penetrates deeper destroying deep-lying hair layers with simultaneous increase of the hair temperature to that at which the coagulation takes place. As the packet duration is commensurable with the hair time of thermal relaxation, then the part of pulse energy consumed for the hair heating up has no time to be transferred to surrounding tissue. As a result, both mechanical and thermal skin damage is minimized what altogether with the increase of depilation effectiveness renders the method used by the apparatus least traumatic.

The comparison of the proposed apparatus with the prior art allows to distinguish the following features:
- The radiation of a nanosecond duration is applied in the form of a packet of pulses with determined energy density and duration.
- The said packet presents itself as a sequence of not less than 5 pulses.
- The hair is irradiated by at least one packet.

All the aforesaid allows to make a conclusion as to the conformity of the claimed technical solution to the criterion "New in the Art".

In spite of the fact that in the medical practice there are known various apparatus for laser depilation the proposed apparatus allows to obtain new results. It has become possible owing to the fact that when using pulses of a nanosecond duration, firstly, the effectiveness of absorption of radiation by hair structures increases what leads to the greater heating up of the hair and, secondly, as a result of radiation absorption inside the hair shaft the microexplosions occur which bring about the mechanical destruction of the hair. The more effective heating up of the hair is due to the fact that during the time of influence by a single pulse (units of a nanosecond) the heat is not able to be transferred to surrounding tissue. When using a sequence of nanosecond pulses each of the pulses of a packet renders the hair partially coagulated and inflicts mechanical damage to it. As the packet duration is commensurable with the hair thermal relaxation time then practically all absorbed energy will be consumed for the heating up of the hair and for the forming of acoustic waves bringing about the hair mechanical destruction. Besides, the said radiation is able to penetrate deeper layers of the hair and provoke the destruction of the follicle.

It is achieved owing to the fact that the first pulse of a packet destroys the absorption centers situated in the upper layer of a hair and makes the said layer "transparent" for the influence of the next pulse from the packet which, in its turn, penetrates deeper causing the destruction of more profound hair layers. As a result, each subsequent pulse of the packet penetrates still deeper causing the hair destruction upon all its length.

All the abovesaid allows to make the conclusion as to the conformity of the claimed technical solution to the criterion "Inventive Level".

The further increment of energy density, duration of a packet application, duration of each pulse and the quantity of pulses in the packet provoke additional overheating or increase of mechanical damages.

### Industrial applicability

The skin is influenced by laser radiation in the form of a pulses packet comprising, for instance, 15 subsequent pulses. The pulses energy in the packet is 3 Joules, each pulse duration is 5 ns, duration of the applied packet is 1,2 ms. To obtain a packet of a pulsed laser radiation with the required quantity of pulses a solid body YAG:Nd laser operating in the Q-generation mode, with wavelength of 1064 nm and a spot diameter of 6 mm may be used.

The influence is carried out by discrete scanning with irradiation of each skin segment comprising one hair and defined by the spot size with one packet.

The employment of the claimed apparatus allows to eliminate overheating and mechanical damage of surrounding tissue.

## Claims

1. An apparatus of laser depilation, comprising means for subjecting the skin to the influence of a pulsed laser radiation of nanosecond duration **characterized in that** the means is adapted to apply the radiation in the form of a packet of pulses with energy density from 3 to 60 Joules/cm² and a packet duration from 0.5 to 10 ms.

2. An apparatus as set forth in claim 1, **characterized in that** said packet of pulses is formed from at least 5 subsequent pulses.

3. An apparatus as set forth in claim 1or 2, **characterized in that** each hair can be irradiated by at least one packet.

4. An apparatus as set forth in one of claims 1 to 3 **characterized in that** the apparatus includes a YAG:Nd laser.

## Patentansprüche

1. Vorrichtung zum Laser-Haarentfernen mit Mitteln, die die Haut dem Einfluss einer gepulsten Laserstrahlung von Nanosekundenlänge aussetzen,
**dadurch gekennzeichnet,**
**dass** die Mittel derart ausgebildet sind, dass die Strahlung in Form eines Pulspakets mit einer Energiedichte von 3-60 Joule/cm² und einer Paketdauer von 0,5-10ms abgegeben wird.

2. Vorrichtung nach Anspruch 1,
**dadurch gekennzeichnet,**
**dass** das Pulspaket aus mindestens fünf aufeinanderfolgenden Impulsen gebildet ist.

3. Vorrichtung nach Anspruch 1 oder 2,
**dadurch gekennzeichnet,**
**dass** jedes Haar durch mindestens ein Pulspaket bestrahlt werden kann.

4. Vorrichtung nach einem der Ansprüche 1 bis 3,
**dadurch gekennzeichnet,**
**dass** die Vorrichtung einen YAB:Nd-Laser aufweist.

## Revendications

1. Appareil d'épilation laser, comprenant des moyens pour soumettre la peau à l'influence d'un rayonnement laser pulsé d'une durée de quelques nanosecondes, **caractérisé en ce que** les moyens sont conçus pour appliquer le rayonnement sous la forme d'un paquet d'impulsions ayant une densité d'énergie de 3 à 60 Joules/cm² et une durée du paquet de 0,5 à 10 ms.

2. Appareil suivant la revendication 1, **caractérisé en ce que** le paquet d'impulsions est formé d'au moins 5 impulsions successives.

3. Appareil suivant la revendication 1 ou 2, **caractérisé en ce que** chaque poil peut être soumis au rayonnement d'au moins un paquet.

4. Appareil suivant l'une des revendications 1 à 3, **caractérisé en ce que** l'appareil comprend un laser YAG:Nd.
